# EUROPÄISCHE PATENTANMELDUNG

(11) **EP 2 308 834 A1**
(43) Veröffentlichungstag der Anmeldung: **13.04.2011**
(21) Anmeldenummer: 09172246.2
(22) Anmeldetag: 05.10.2009
(51) Int. Cl.: C07C 253/34, C07C 255/25

(54) **Verfahren zur Isolierung von Methylglycinnitril-N,N-diacetonitril**

(71) Anmelder: BASF SE, 67056 Ludwigshafen (DE)
(72) Erfinder: Judat, Bernd, Dr., 67067 Ludwigshafen (DE); Oftring, Alfred, Dr., 67098 Bad Dürkheim (DE); Stamm, Armin, Dr., 55268 Nieder-Olm (DE); Teich, Friedhelm, Dr., 68535 Neckarhausen (DE)

(57) **Zusammenfassung**

Die vorliegende Erfindung betrifft ein Verfahren zur Isolierung von Methylglycinnitril-N,N-diacetonitril (MGDN) aus einem MGDN enthaltenden, wässrigen Gemisch, umfassend die Abkühlung des wässrigen Gemisches in einem oder mehreren Schritten, wobei in einem dieser Schritte das Gemisch mit einer Abkühlrate von mindestens 20K/h von einer Temperatur oberhalb des Erstarrungspunktes von MGDN auf eine Temperatur unterhalb des Erstarrungspunktes von MGDN abgekühlt wird und wobei das Verfahren kontinuierlich ausgeübt wird.

## Beschreibung

Die vorliegende Erfindung betrifft ein Verfahren zur Isolierung von Methylglycinnitril-N,N-diacetonitril (MGDN) aus einem MGDN enthaltenden, wässrigen Gemisch.

Bevorzugte Ausführungsformen sind den Unteransprüchen und der Beschreibung zu entnehmen.

Die häufig als Komplexbildner, zum Beispiel in Haushaltsreinigungsmitteln eingesetzten Aminopolyphosphonate, Polycarboxylate oder Aminopolycarboxylate, wie Ethylendiaminotetraessigsäure (EDTA), sind nur in geringem Maße biologisch abbaubar. Eine preiswerte Alternative stellen Glycin-N,N-Diessigsäurederivate wie Methylglycin-N,N-Diessigsäure (MGDA) dar, welche ungiftig und gut biologisch abbaubar sind. Die Verwendung von MGDA und von verwandten Glycin-N,N-Diessigsäurederivaten in Reinigungsmitteln sowie deren Synthesen sind zum Beispiel in WO-A 94/29421 und US 5,849,950 beschrieben. Für eine kostengünstige Produktion der Glycin-N,N-Diessigsäurederivate werden hohe Anforderungen an die Ausbeute der einzelnen Syntheseschritte und Reinheit der isolierten Zwischenprodukte gestellt.

MGDA wird im Allgemeinen durch Umsetzen von Iminodiacetontril (IDN) mit Acetaldehyd und Blausäure oder von alpha-Alaninnitril mit Formaldehyd und Blausäure (sogenannte pH-kontrollierte Strecker Reaktion) erhalten, wobei das als Zwischenprodukt erhaltene MGDN in einem weiteren Schritt einer alkalischen Hydrolyse mit Natronlauge unterworfen wird. Hierdurch wird das Trinatriumsalz von MGDA erhalten. Um hohe MGDA-Ausbeuten zu erzielen ist es wünschenswert, MGDN als Zwischenprodukt zu isolieren und als Reinstoff in dem sich anschließenden Hydrolyseschritt einzusetzen.

MGDN kann auf verschiedene Arten hergestellt werden. In der Regel fällt MGDN bei der Synthese als wässriges Gemisch an. Es ist möglich, dass derartige Gemische ausschließlich aus Wasser und MGDN bestehen. Wässrige MGDN enthaltende Gemische können aber außerdem eine Reihe von Nebenkomponenten umfassen. Wird MGDN zum Beispiel durch pH-kontrollierte Strecker-Reaktion hergestellt, kann Iminodiacetonitril (IDN) entweder als kristalliner Rohstoff eingesetzt oder aber durch pH-kontroiiierte Umsetzung von Urotropin mit HCN in wässriger Lösung erzeugt und ohne selbst isoliert zu werden zum MGDN umgesetzt werden. Als Nebenkomponenten enthält das wässrige MGDN Gemisch dann unter anderem Ammoniumsulfat, Acetaldehydcyanhydrin, Formaldehydcyanhydrin, Methylen-bis-iminodiacetonitril (MBIDN), Nitrilotriacetonitril (NTN), Di-Methylglycinacetonitril (DMGN), Acetaldehyd, Blausäure sowie nicht umgesetzte Edukte.

MGDN weist eine stark temperaturabhängige Löslichkeit in Wasser auf. So lassen sich bei 10 °C nur noch etwa 0,5 Gew.-% MGDN in Wasser oder einer wässrigen Ammoniumsulfatlösung lösen. Bei ca. 60 °C beträgt die Löslichkeit noch etwa 5 Gew.-%. Oberhalb von ca. 60 °C liegt eine Emulsion aus MGDN und seiner wässrigen Lösung mit mehr als 5 Gew.-% gelöstem MGDN vor. Ein vollständiges Phasendiagramm des Systems Wasser-MGDN ist nicht bekannt.

MGDN weist einen Erstarrungspunkt auf, der allerdings nicht konstant ist, sondern beispielsweise von allen im Gemisch vorliegenden Komponenten abhängt wie zum Beispiel vom Salzgehalt des Gemisches, in dem MGDN vorliegt, von der Konzentration von MGDN im Solvens und von der Art und Menge der im Gemisch enthaltenen Nebenprodukte. Der Erstarrungspunkt von reinem MGDN beträgt ca. 82 °C, der Erstarrungspunkt von MGDN in wässrigen Gemischen liegt in der Regel zwischen 55 und 65 °C.

Aufgrund der starken Temperaturabhängigkeit der Löslichkeit von MGDN in Wasser kann MGDN durch Kühlungskristallisation und anschließender Fest/Flüssig-Trennung aus wässrigen Gemischen isoliert werden. Beim Abkühlen einer wässrigen Lösung oder Emulsion von MGDN fällt MGDN als Feststoff in Form von feinen, nadeligen Kristallen aus, welche agglomerieren können. Eines der Probleme bei der Kristallisation von MGDN ist der Einschluss von Verunreinigungen.

In US 5,849,950, Beispiel 2, wird MGDN aus dem Rohproduktgemisch der Umsetzung von HCN, Formaldehyd und Alaninnitril, welches in einem vorgelagerten Schritt in situ aus Acetaldehyd, HCN und Ammoniak erzeugt wird, durch Kühlung des Produktgemischs auskristallisiert.

In EP 1883623 wird ein Verfahren zur Isolierung von MGDN beschrieben, bei dem eine wässrige Emulsion von MGDN in mindestens zwei Schritten abgekühlt wird. Der Abkühlungsschritt von oberhalb nach unterhalb des Erstarrungspunktes von MGDN geschieht dabei sehr langsam mit einer mittleren Abkühlungsrate von weniger als 5 K/h, um eine langsame Kristallisation des MGDN zu erreichen.

Aufgabe dieser Erfindung war es, ein verbessertes Verfahren zur Abtrennung von MGDN aus den bei seiner Herstellung anfallenden wässrigen Rohproduktgemischen bereitzustellen, das hohe Ausbeuten und Reinheiten an MGDN ermöglicht. Darüber hinaus sollte ein Verfahren zur Verfügung gestellt werden, dass eine möglichst konstante Qualität des Produktes ergibt. Des Weiteren war das Ziel, eine möglichst hohe Raum-Zeit-Ausbeute zu erreichen.

Die Aufgabe wurde gelöst durch das oben genannte Verfahren, umfassend die Abkühlung des wässrigen, MGDN haltigen Gemisches in einem oder mehreren Schritten, wobei in einem dieser Schritte das Gemisch mit einer Abkühlrate von mindestens 20 K/h von einer Temperatur oberhalb des Erstarrungspunktes von MGDN auf eine Temperatur unterhalb des Erstarrungspunktes von MGDN abgekühlt wird und wobei das Verfahren kontinuierlich ausgeübt wird.

Die Isolierung von Bestandteilen aus einem flüssigen Gemisch durch Bildung von Ausfällungen oder Niederschlägen wird durch den Fachmann häufig als "Kristallisation" bezeichnet. Die Begriffe "Kristalle", "Kristallisation" und "kristallin" im Sinne dieser Erfindung beziehen sich daher nicht immer nur auf Stoffe, deren Moleküle regelmäßig im Kristallgitter angeordnet sind, sondern umfassen allgemein alle Feststoffe und Niederschläge, die sich aus den beschriebenen wässrigen Gemischen abscheiden, auch wenn diese amorphe Bereiche aufweisen. Je nach Randbedingungen kann es sich zum Beispiel auch um ein Erstarren eines Flüssigkeitstropfens handeln.

Als kontinuierliches Verfahren wird im Sinne dieser Erfindung eine Kristallisation verstanden, bei dem über einen längeren Zeitraum, zum Beispiel über mehrere Stunden, Tage, Wochen oder Jahre, ein Massenstrom von MGDN-reichem Rohgemisch in die Vorrichtung, in der das erfindungsgemäße Verfahren durchgeführt wird, zugeführt wird, ohne dass die Kristallisation unterbrochen oder der Kristallisator beziehungsweise die Kristallisatoren entleert werden müssten. Gleichzeitig wird bei einem kontinuierlichen Verfahren über den selben Zeitraum ein im Mittel im Wesentlichen gleich großer beziehungsweise gleich großer Massenstrom an abgekühltem Endgemisch aus dem Kristallisator entnommen. Die Massenströme können in ihrer Menge pro Zeiteinheit über den Zeitraum konstant sein oder variieren. Unter variieren ist auch zu verstehen, dass die Massenströme für kurze Zeit unterbrochen werden, zum Beispiel durch getaktetes Öffnen und Schließen von Ventilen. Nach einer der bevorzugten Ausführungsformen sind die Massenströme im Wesentlichen konstant.

Als Kristallisatoren werden die apparativen Vorrichtungen verstanden, in denen die unten beschriebenen Abkühlungsschritte des wässrigen MGDN haltigen Gemisches durchgeführt werden.

Erfindungsgemäß wird die Isolierung von MGDN aus dem wässrigen Gemisch in einem oder in mehreren Abkühlungsschritten durchgeführt. Als ein Abkühlungsschritt wird der Vorgang verstanden, bei dem die Temperatur des wässrigen Gemisches von einer Anfangstemperatur auf eine Endtemperatur erniedrigt wird. Die Differenz der beiden Temperaturen kann zum Beispiel mindestens ein Kelvin betragen, meist beträgt sie mindestens zwei Kelvin. In einer bevorzugten Variante beträgt die Temperaturdifferenz mindestens fünf oder mindestens zehn Kelvin. In der Regel umfasst das erfindungsgemäße Verfahren einen bis fünf Abkühlungsschritte, bevorzugt zwei bis drei.

Im Verlaufe eines Abkühlungsschrittes verändert sich das wässrige Gemisch in seiner Zusammensetzung. Vor einem Abkühlungsschritt ist das wässrige Gemisch wärmer und es liegt eine größere Menge an MGDN im wässrigen Gemisch gelöst oder emulgiert vor. Das wässrige Gemisch in seinem Zustand vor einem Abkühlungsschritt wird in dieser Beschreibung auch als "Rohgemisch" bezeichnet. Im Gegensatz dazu wird das wässrige Gemisch nach einem Abkühlungsschritt auch als "Endgemisch" bezeichnet. Dieses ist kälter und enthält nur noch eine kleinere Menge MGDN in gelöster Form.

Ist der Erstarrungspunkt von MGDN für ein bestimmtes wässriges Gemisch nicht bekannt, so kann er zum Beispiel dadurch bestimmt werden, dass man durch mikroskopische Beobachtung die Temperatur bestimmt, bei der ein in Wasser emulgierter MGDN Tropfen fest wird. Einer der Abkühlungsschritte beinhaltet erfindungsgemäß den Phasenübergang, in Wasser emulgierten MGDN von flüssig nach fest am Erstarrungspunkt. Dieser Abkühlungsschritt ist von besonderer Bedeutung für das Verfahren, denn während dieses Abkühlungsschrittes kristallisiert der größte Anteil des MGDN. Die Anfangstemperatur für diesen Abkühlungsschritt liegt in der Regel mindestens 0,5 K über dem Erstarrungspunkt, bevorzugt mindestens ein Kelvin, besonders bevorzugt mindestens zwei Kelvin, ganz besonders bevorzugt mindestens fünf Kelvin, insbesondere bevorzugt mindestens 10 Kelvin. Im Allgemeinen kann die Temperatur des Rohgemisches beliebig hoch über dem Erstarrungspunkt liegen. In der Regel liegt sie jedoch nicht oberhalb des Siedepunktes des Rohgemisches bei Atmosphärendruck, da sonst das erfindungsgemäße Verfahren unter erhöhtem Druck ausgeführt werden müsste. Bevorzugt liegt sie nicht höher als 90°C. Die Endtemperatur des Abkühlungsschrittes liegt in der Regel mindestens 0,5 K unterhalb dem Erstarrungspunkt von MGDN, bevorzugt mindestens 1 K, besonders bevorzugt mindestens 2 K, ganz besonders bevorzugt mindestens 5 K. Es ist ebenfalls möglich, eine Endtemperatur von mehr als 10 K unterhalb des Erstarrungspunktes zu wählen.

Dem Abkühlungsschritt über den Erstarrungspunkt können beliebig viele Abkühlungsschritte vor und nach geschaltet werden. Vorgeschaltete Abkühlungsschritte haben in der Regel die Annäherung der Temperatur des Rohgemisches an den Erstarrungspunkt zum Zweck. Nach geschaltete Abkühlungsschritte dienen in erster Linie der Steigerung der Ausbeute durch die Erniedrigung der Löslichkeit von MGDN in Wasser.

Für die nach geschalteten Abkühlungsschritte ist die Endtemperatur des vorhergehenden Schritts bevorzugt die Anfangstemperatur des nachfolgenden Schrittes. Die Temperaturdifferenz der nach geschalteten Abkühlungsschritte beträgt in der Regel mehr als 10 K, bevorzugt mehr als 20 K und besonders bevorzugt mehr als 30 K. In einer insbesondere bevorzugten Ausführungsform beträgt die Temperaturdifferenz dieser Abkühlungsschritte 40 bis 50 K.

In weniger bevorzugten Ausführungsformen des erfindungsgemäßen Verfahrens kann das wässrige Gemisch nach einem Abkühlungsschritt aber auch anderen Schritten unterworfen werden und zum Beispiel erneut erwärmt werden. Das Ausführen von Impfschleifen ist für das erfindungsgemäße Verfahren weniger bevorzugt.

In einer bevorzugten Ausführungsform wird die Kristallisation in zwei Abkühlungsschritten durchgeführt, wobei das Rohgemisch im ersten Schritt von einer Temperatur oberhalb des Erstarrungspunktes von MGDN auf eine Temperatur unterhalb dieser Temperatur abgekühlt wird. In einem zweiten Schritt wird die Temperatur dann noch einmal deutlich erniedrigt, um die Ausbeute zu steigern.

In der Regel beträgt die Endtemperatur des letzten Abkühlungsschrittes von -10 °C bis 50°C, bevorzugt von 0 °C bis 30°C und besonders bevorzugt von 5 °C bis 15°C.

Als Abkühlungsgeschwindigkeit im Sinne dieser Erfindung wird jeweils der mittlere Temperaturgradient über die Zeit von der Anfangstemperatur bis zur Endtemperatur eines Abkühlungsschrittes verstanden. Erfindungsgemäß beträgt die Abkühlungsgeschwindigkeit während des Abkühlungsschrittes von oberhalb nach unterhalb des Erstarrungspunktes von MGDN mindestens 20 K/h, bevorzugt mindestens 50 K/h, besonders bevorzugt mindestens 100 K/h. Es ist möglich, alle Abkühlungsschritte mit derselben Abkühlungsgeschwindigkeit durchzuführen, sie können sich aber auch in jedem Abkühlungsschritt unterscheiden.

In einer bevorzugten Ausführungsform der Erfindung erfolgt die Abkühlung des Rohgemisches derart, dass das Rohgemisch zu wässrigem Gemisch zugegeben wird, welches die Endtemperatur oder eine Temperatur leicht darunter aufweist. Bevorzugt ist das Volumen des vorgelegten MGDN enthaltendem, wässrigen Gemisches groß gegenüber dem Massenstrom an Rohgemisch. Eine solche Ausführungsform kann in Abkühlgeschwindigkeiten von über 1000 K/h resultieren, auch Abkühlgeschwindigkeiten von über 3000 K/h, 5000 K/h oder 7500 K/h können so realisiert werden.

Die Art und Weise, in der die Kühlung der Kristallisatoren durchgeführt wird, ist erfindungsgemäß nicht entscheidend. Mögliche Methoden der Kühlung sind zum Beispiel die Kühlung über Wärmetauscher oder Verdampfen von Wasser aus dem wässrigen Gemisch. Eine Kühlung der Kristallisatoren über Wärmetauscher kann den Vorteil bieten, dass sich wenig Schaum in den Kristallisatoren bildet. Verdampfen von Wasser aus dem wässrigen Gemisch kann den Vorteil bieten, dass sich weniger Kristallkeime und weniger Belag an den Wänden des Kristallisators bilden. Das Verdampfen von Wasser kann bei konstanter Wassermenge geschehen, indem das verdampfte Wasser unter Rückfluss wieder zurückfließt. Alternativ kann Wasser auch unter Verringerung der Wassermenge aus dem wässrigen Gemisch entfernt werden und so zu einer Aufkonzentration des MGDN im wässrigen Gemisch führen. Die Methoden können auch miteinander kombiniert werden.

Die Abkühlung kann auch dadurch erfolgen, dass das wässrige Gemisch in einem Rohrkristallisator, der entlang der Fließstrecke verschiedene Temperaturzonen aufweist, abgekühlt wird. Rohrkristallisatoren können mit oder ohne Kratzelemente betrieben werden, die die Wände des Kristallisators von Belägen freihalten. Eine weitere Möglichkeit ist die Verwendung von Kühlscheiben (engl.: cooling discs). Derartige Kühlmethoden sind dem Fachmann an sich bekannt.

Bevorzugt wird während des Abkühlungsschrittes von oberhalb nach unterhalb des Erstarrungspunktes von MGDN mechanische Energie eingetragen. In einer bevorzugten Ausführungsform wird während jedes Abkühlungsschrittes mechanische Energie eingetragen. Es ist auch möglich, während des gesamten Verfahrens zur Isolierung von MGDN mechanische Energie einzutragen. In der Regel wird mindestens eine Energie mit einem mittleren spezifischen Energieeintrag von 0,5 kW/m³ eingetragen, bevorzugt mindestens 0,8 kW/m³, besonders bevorzugt mindestens 1 kW/m³ und insbesondere bevorzugt 1,5 kW/m³. Der Eintrag an Energie ist prinzipiell nicht nach oben begrenzt, wird sich aber nach apparativen Vorraussetzungen richten und nach praktischen Erwägungen wie zum Beispiel der Größe und Konstruktion des Kristallisators oder ggf. nach der Art des Rührers, nach wirtschaftlichen Erwägungen und nach der angestrebten Kristallgröße. Dadurch, dass mittels des erfindungsgemäßen Verfahrens mechanische Energie eingetragen wird, wird nur wenig wässriges Gemisch, dass sämtliche Nebenkomponenten der MGDN Herstellung enthalten kann, als Verunreinigung in Kristallagglomeraten eingeschlossen. Im Gegensatz zu Kristallagglomerate enthaltenden Kristallisaten aus anderen Verfahren, weist MGDN, welches nach dem erfindungsgemäßen Verfahren isoliert worden ist, eine reduzierte bräunliche Färbung auf. Es ist ein überraschendes Ergebnis des erfindungsgemäßen Verfahrens, dass die Bildung von Kristallagglomeraten trotz der hohem Abkühlgeschwindigkeiten effektiv eingeschränkt werden kann, denn die im Gemisch emulgierten flüssigen MGDN Tropfen haben eine hohe Viskosität und Klebrigkeit. Durch das erfindungsgemäße Verfahren wird außerdem die Neigung zur Ablagerung der emulgierten MGDN Tropfen auf Oberflächen und zur Bildung von Verkrustungen an den Wänden des Kristallisators vermindert.

Durch geeignete Einstellung des mechanischen Energieeintrags kann außerdem die Größe der sich bildenden festen MGDN Partikel so gesteuert werden, dass die erhaltenen Partikel groß genug sind, um sie gut filtrieren zu können, jedoch klein genug, damit sie sich für folgende Umsetzungen wieder gut in dem betreffenden Reaktionsmedium auflösen. Im Allgemeinen sind Partikel mit mittleren Größen im Bereich von 100 bis 1000 µm gut handhabbar. Partikel, die kleinere oder größere mittlere Größen aufweisen, können ebenfalls hergestellt werden. Sie erfordern gegebenenfalls besondere apparative Handhabung. Methoden zur Bestimmung der Partikelgrößen sind dem Fachmann bekannt.

Zudem beeinflusst der Energieeintrag das Ausmaß der Belagbildung an den Wänden. Eine exakte Korrelation zum Beispiel bestimmter Partikelgrößen mit bestimmten mechanischen Energien ist nicht möglich, da dieses stark von den apparativen Vorraussetzungen und den anderen Randbedingungen, insbesondere der Zusammensetzung des Rohgemisches, abhängt. In der Regel bedingt der Eintrag einer hohen mechanischen Energie tendenziell kleinere Partikel und tendenziell weniger Belagbildung. Es ist nicht entscheidend, auf welche Weise die Energie in das System eingetragen wird. Eine mögliche Energiequelle ist zum Beispiel Ultraschall oder Verdüsen des wässrigen Gemisches. Bevorzugt geschieht der Energieeintrag durch mechanisches Rühren. Wird der Großteil der mechanischen Energie durch Rühren eingetragen, sind hierfür insbesondere schnell laufende Rührertypen wie Propellerrührer, Schrägblattrührer, Scheibenrührer oder Zahnscheibenrührer bevorzugt. Bevorzugt wird durch den Rührvorgang im Kristallisator eine turbulente Strömung erzeugt. Je nach Kristallisatorkonstruktion kann es sinnvoll sein, Stromstörer zu verwenden.

Die einzelnen Abkühlungsschritte können in verschiedenen Typen von Kristallisatoren durchgeführt werden. Deren Art ist für das Verfahren nicht entscheidend. Mögliche Kristallisatorarten sind zum Beispiel Zwangsumlauf-, Leitrohr- oder Rührkessel-Kristallisatoren. Letztere können Einbauten aufweisen, bevorzugt werden sie jedoch frei von Einbauten verwendet.

In einer bevorzugten Ausführungsform wird das erfindungsgemäße Verfahren dergestalt ausgeführt, dass jeder Abkühlungsschritt in einem Rührkessel als Kristallisator durchgeführt wird, in den ein kontinuierlicher Strom von MGDN Rohgemisch zugegeben wird und ein kontinuierlicher Strom von abgekühltem, an MGDN verarmten Gemisch abgeführt wird. Ganz besonders bevorzugt wird das erfindungsgemäße Verfahren in einer Rührkesselkaskade durchgeführt. Insbesondere bevorzugt umfasst die Rührkesselkaskade zwei Rührkessel.

Die Zugabe des Rohgemisches kann prinzipiell an jeder Stelle des Kristallisators erfolgen. Bevorzugt geschieht dieses jedoch auf eine Weise, dass das zugegebene Rohgemisch sofort hohe Scherkräfte erfährt. So ist die Zugabe durch ein Ventil direkt in das vorlegte wässrige Gemisch oder durch getauchte Zugabe, zum Beispiel über einen gegebenenfalls vorhandenen Rührer, eine Möglichkeit. Weniger bevorzugt ist das Zutropfen von oben auf die Oberfläche des vorgelegten wässrigen Gemisches. Der Abfluss des Endgemisches kann prinzipiell an jeder Stelle des Kristallisators passieren. Bevorzugt wird das abgekühlte Endgemisch an der Unterseite des Kristallisators entnommen. Die mittlere Verweilzeit des wässrigen Gemisches im Kristallisator beträgt in der Regel mindestens 10 Minuten, bevorzugt 20 Minuten, ganz besonders bevorzugt 30 Minuten und insbesondere bevorzugt mindestens 60 Minuten. In einer bevorzugten Variante umfasst das Verfahren zwei Abkühlungsschritte, die jeweils in einem Rührkessel durchgeführt werden.

Die Abtrennung des festen MGDN vom wässrigen Gemisch kann nach dem Fachmann bekannten Verfahren geschehen, zum Beispiel mittels eines Hydrozylinders zur fest/flüssig Abtrennung oder einem Bandfilter. Es ist nach jedem Abkühlungsschritt möglich, kristallisiertes MGDN vom wässrigen Gemisch abzutrennen. Es ist ebenso möglich, kristallisiertes MGDN erst nach dem letzten Abkühlungsschritt abzutrennen. Die Abtrennung kann kontinuierlich oder diskontinuierlich geschehen.

Das erfindungsgemäße Verfahren eignet sich zur Isolierung von MGDN aus einem wässrigen Gemisch, unabhängig davon, auf welche Weise dieses hergestellt worden ist. Es ist möglich, das wässrige Gemisch durch Mischen von festem MGDN mit Wasser bereit zu stellen. Bevorzugt wird das wässrige Gemisch erhalten, indem man MGDN im wässrigen Medium in situ herstellt, gegebenenfalls das Verhältnis von MGDN zu Lösemittel anpasst und mit diesem wässrigen Gemisch das erfindungsgemäße Verfahren zur Isolierung von MGDN ausführt.

Das MGDN enthaltende wässrige Gemisch, aus dem mit dem erfindungsgemäßen Verfahren MGDN isoliert wird, kann zum Beispiel erhalten werden durch
1. Umsetzung von Iminodiacetonitril (IDN) mit HCN und Acetaldehyd in wässriger Lösung. Iminodiacetonitril kann in einer vorgelagerten Stufe aus Urotropin und Blausäure oder aus Formaldehydcyanhydrin und Ammoniak als wässrige Emulsion erhalten werden; oder
2. Umsetzung von Alaninnitril, mit HCN und Formaldehyd in wässriger Lösung. Alaninnitril kann in einer vorgelagerten Stufe aus Acetaldehyd, HCN und Ammoniak oder Acetaldehydcyanhydrin und Ammoniak erhalten werden.

Die Bedingungen, unter denen MGDN enthaltendes wässriges Gemisch erhalten werden kann, sind dem Fachmann zum Beispiel aus EP 1883623 bekannt.

Das wässrige, MGDN haltige Gemisch enthält vor der Abkühlung unter den Erstarrungspunkt von MGDN bevorzugt 5 bis 50 Gew% MGDN, besonders bevorzugt 10 bis 40 Gew% und ganz besonders bevorzugt 15 bis 30 Gew% MGDN. MGDN liegt zum Teil gelöst vor. Übersteigt der MGDN Gehalt des wässrigen Gemisches dessen Löslichkeit, liegt MGDN zum Teil als Emulsion vor.

Das Lösungsmittel des wässrigen Gemisches besteht im Wesentlichen aus Wasser. In der Regel sind mindestens 70 Gew% des verwendeten Lösungsmittels Wasser, bevorzugt mindestens 85 Gew%, besonders bevorzugt 95 Gew%. In einer ganz besonders bevorzugten Ausführungsform enthalt das wässrige Gemisch außer Wasser keine weiteren Lösungsmittel.

In weniger bevorzugten Ausführungsformen der Erfindung enthält das wässrige Gemisch neben Wasser noch organische Lösungsmittel. Diese müssen mit Wasser unter den Verfahrensbedingungen mischbar sein. Geeignete organische Lösungsmittel sind zum Beispiel Alkohole wie Methanol oder Ethanol oder andere polare Lösungsmittel. Durch das erfindungsgemäße Verfahren gelingt es in der Regel, MGDN mit einer Ausbeute von mindestens 70% zu isolieren. Bevorzugt werden mindestens 85%, besonders bevorzugt 90% und ganz besonders bevorzugt 95% des eingesetzten MGDNs isoliert, bezogen auf den Gehalt von MGDN in der wässrigen Mischung vor dem ersten Abkühlungsschritt.

Nach der Isolierung kann festes oder wieder gelöstes MGDN weiteren Reinigungsschritten unterworfen werden. Es ist möglich die Farbzahl des MGDN durch Adsorption an Aktivkohle oder Bleichprozesse zu vermindern. Mögliche Bleichprozesse umfassen zum Beispiel Photobleiche, Photooxidation oder Ozonolyse. Derartige Bleichprozesse sind dem Fachmann an sich bekannt.

Die Reinheit des isolierten MGDN beträgt in der Regel mehr als 98 Gew%.

Ein besonderer Vorteil des erfindungsgemäßen Verfahrens ist eine sehr hohe Raum-Zeit Ausbeute.

### Beispiele

Die folgenden Beispiele sollen die Eigenschaften dieser Erfindung erläutern, ohne sie aber einzuschränken.

Als "Teile", Prozent oder ppm werden in dieser Schrift, wenn nicht anders angegeben, Gewichtsanteile verstanden.

### Beispiel 1: Herstellung eines wässrigen MGDN Rohgemisches

IDN wurde durch Umsetzung einer Lösung von 173,9 g (1,241 mol) Urotropin in 535 g Wasser mit 210,9 g (7,814 mol) Blausäure hergestellt. Zur Regelung des pH-Wertes auf 5,8 - 5,9 wurden insgesamt 188 g 50 Gew%ige Schwefelsäure zudosiert. Es resultierten 1090 g einer wässrigen Lösung, die 336,3 g (3,54 mol) IDN enthielt (95% Ausbeute bezogen auf Formaldehyd). Der pH-Wert der Lösung wurde durch Zugabe von 60 g (0,306 mol) 50 gew.-%iger Schwefelsäure auf 1,8 und ihre Temperatur auf 60°C eingestellt. Anschließend wurden innerhalb von 75 Minuten 105,1 g (3,894 mol) HCN und 171,6 g (3,894 mol) Acetaldehyd zudosiert. Die Temperatur stieg auf ca. 80°C. Es wurde noch 60 Minuten bei 80°C weitergerührt. Der pH-Wert fiel während der Zugabe der Reaktanden und während der Nachreaktion auf ca. 1,2. Es resultierten ca. 1440 g einer ca. 36 gew.-%ige MGDN-Emulsion, welche 515 g MGDN enthielt (3,469 mol; 98% Ausbeute bezogen auf IDN). Das erhaltene MGDN Rohgemisch wies eine dunkelbraune Farbe auf.

### Beispiele 2 bis 3: Kontinuierliche zweistufige Kristallisation von MGDN

Für die folgenden Beispiele wurde MGDN Romemulsion benutzt, die entsprechend Beispiel 1 hergestellt worden war.

Für die Versuche wurde eine Versuchsanlage aufgebaut, die aus zwei Rührkesseln bestand. Aus einer gerührten und auf 70°C temperierten Vorlage wurde die MGDN Rohlösung kontinuierlich in den ersten Rührkessel gepumpt. Das Überführen in den zweiten Rührkessel erfolgte standgeregelt durch getaktetes Öffnen und Schließen des Bodenablassventils des ersten Rührkessels. Um Verkrustungen im Ablaufrohr zu vermeiden, wurde nach jedem Öffnen des Bodenablassventils und Ablauf der Suspensionsportion in den zweiten Rührkessel ein Spülzyklus mit warmem Wasser durchgeführt. Die Spüllösung wurde über einen Dreiwegehahn aus dem System ausgeschleust. Durch diese Maßnahme war ein kontinuierlicher Betrieb der zweistufigen Anlage möglich.

### Beispiel 2: Kontinuierliche zweistufige Kristallisation von MGDN

Wässriges MGDN Gemisch mit einem MGDN Gehalt von 21,0 Gew% wurde vorgelegt und auf eine Temperatur von 55°C aufgeheizt. Das Gemisch wurde mit einem Propellerrührer mit 720 Umdrehungen pro Minute gerührt. Zu diesem Gemisch wurde über einen Zeitraum von circa sechs Stunden kontinuierlich wässriges MGDN Rohgemisch mit einer Temperatur von 70°C zudosiert. Nach ca. fünf Stunden hatte sich ein stationärer Zustand eingestellt. Die durchschnittliche Verweilzeit betrug ca. eine Stunde. Es bildeten sich schlagartig große Kristalle von MGDN.

Das wässrige MGDN Gemisch wurde wie oben beschrieben aus dem Rührkessel entfernt und in einen zweiten, identischen Rührkessel geleitet, welcher ebenfalls wässriges MGDN Gemisch enthielt und auf 13°C thermostatiert war. Aus diesem Gefäß wurde erneut standgeregelt wässriges MGDN Gemisch entnommen.

Aus dem wässrigen Gemisch wurde kristallisiertes MGDN durch Druckfiltration abgetrennt und mit Wasser gewaschen.

Die Ausbeute an kristallinem MGDN betrug über 95%, bezogen auf das in dem anfangs eingesetzten wässrigen Gemisch enthaltene MGDN.

Das so erhaltene kristalline MGDN wurde per HPLC analysiert. Der Gehalt an IDN betrug weniger als 0,01 % und lag unterhalb der Nachweisgrenze. Das erhaltene MGDN hatte eine hellbeige Farbe.

### Beispiel 3: Kontinuierliche zweistufige Kristallisation von MGDN

Wässriges MGDN Gemisch mit einem MGDN Gehalt von 19,9 Gew% wurde vorgelegt und auf eine Temperatur von 36°C aufgeheizt. Das Gemisch wurde mit einem Propellerrührer mit 720 Umdrehungen pro Minute gerührt. Zu diesem Gemisch wurde über einen Zeitraum von ca. sechs Stunden kontinuierlich wässriges MGDN Rohgemisch mit einer Temperatur von 70°C zudosiert. Die durchschnittliche Verweilzeit betrug ca. eine Stunde. Es bildeten sich schlagartig große Kristalle von MGDN.

Das wässrige MGDN Gemisch wurde wie oben beschrieben aus dem Rührkessel entfernt und in einen zweiten, identischen Rührkessel geleitet, welcher ebenfalls wässriges MGDN Gemisch enthielt und auf 12°C thermostatiert war. Aus diesem Gefäß wurde erneut standgeregelt wässriges MGDN Gemisch entnommen.

Aus dem wässrigen Gemisch wurde kristallisiertes MGDN durch Druckfiltration abgetrennt und mit Wasser gewaschen.

Die Ausbeute an kristallinem MGDN betrug über 95%, bezogen auf das im Rohgemisch enthaltene MGDN.

Das so erhaltene kristalline MGDN wurde per HPLC analysiert. Der Gehalt der Nebenprodukte IDN betrug weniger als 0,01 % und lag unterhalb der Nachweisgrenze. Das erhaltene MGDN hatte eine hellbeige Farbe.

## Patentansprüche

1. Verfahren zur Isolierung von Methylglycinnitril-N,N-diacetonitril (MGDN) aus einem MGDN enthaltenden, wässrigen Gemisch, umfassend die Abkühlung des Gemisches in einem oder mehreren Schritten, wobei in einem dieser Schritte das Gemisch mit einer Abkühlrate von mindestens 20K/h von einer Temperatur oberhalb des Erstarrungspunktes von MGDN auf eine Temperatur unterhalb des Erstarrungspunktes von MGDN abgekühlt wird und wobei das Verfahren kontinuierlich ausgeübt wird.

2. Verfahren nach mindestens einem der vorstehenden Ansprüche, wobei alle Abkühlungsschritte mit einer Abkühlrate von mindestens 20 K/h durchgeführt werden.

3. Verfahren nach mindestens einem der vorstehenden Ansprüche, wobei während des Abkühlungsschrittes von oberhalb des Erstarrungspunktes von MGDN auf eine Temperatur unterhalb des Erstarrungspunktes von MGDN in das wässrige Gemisch mechanische Energie mit einem mittleren spezifischen Energieeintrag von mindestens 0,5 kW/m³ eingetragen wird.

4. Verfahren nach mindestens einem der vorstehenden Ansprüche, wobei mindestens ein Abkühlungsschritt derart ausgeführt wird, dass das abzukühlende MGDN enthaltende wässrige Gemisch zu kälterem, MGDN enthaltendem wässrigen Gemisch mit einer Temperatur unterhalb des Erstarrungspunktes von MGDN zugegeben wird.

5. Verfahren nach mindestens einem der vorstehenden Ansprüche, wobei die Temperatur des wässrigen Gemisches nach dem letzten Abkühlungsschritt von -10 °C bis 50 °C beträgt.

6. Verfahren nach mindestens einem der vorstehenden Ansprüche, wobei die Abkühlung in zwei Schritten erfolgt.

7. Verfahren nach mindestens einem der vorstehenden Ansprüche, wobei das erhaltene MGDN eine Reinheit von mindestens 90% aufweist.

## Geänderte Patentansprüche

### Geänderte Patentansprüche gemäss Regel 137(2) EPÜ.

**1.** Verfahren zur Isolierung von Methylglycinnitril-N,N-diacetonitril (MGDN) aus einem MGDN enthaltenden, wässrigen Gemisch, umfassend die Abkühlung des Gemisches in einem oder mehreren Schritten, wobei in einem dieser Schritte das Gemisch mit einer Abkühlrate von mindestens 20K/h von einer Temperatur oberhalb des Erstarrungspunktes von MGDN auf eine Temperatur unterhalb des Erstarrungspunktes von MGDN abgekühlt wird und wobei das Verfahren kontinuierlich ausgeübt wird.

**2.** Verfahren nach mindestens einem der vorstehenden Ansprüche, wobei alle Abkühlungsschritte mit einer Abkühlrate von mindestens 20 K/h durchgeführt werden.

**3.** Verfahren nach mindestens einem der vorstehenden Ansprüche, wobei während des Abkühlungsschrittes von oberhalb des Erstarrungspunktes von MGDN auf eine Temperatur unterhalb des Erstarrungspunktes von MGDN in das wässrige Gemisch mechanische Energie mit einem mittleren spezifischen Energieeintrag von mindestens 0,5 kW/m³ eingetragen wird.

**4.** Verfahren nach mindestens einem der vorstehenden Ansprüche, wobei mindestens ein Abkühlungsschritt derart ausgeführt wird, dass das abzukühlende MGDN enthaltende wässrige Gemisch zu kälterem, MGDN enthaltendem wässrigen Gemisch mit einer Temperatur unterhalb des Erstarrungspunktes von MGDN zugegeben wird.

**5.** Verfahren nach mindestens einem der vorstehenden Ansprüche, wobei die Temperatur des wässrigen Gemisches nach dem letzten Abkühlungsschritt von -10 °C bis 50 °C beträgt.

**6.** Verfahren nach mindestens einem der vorstehenden Ansprüche, wobei die Abkühlung in zwei Schritten erfolgt.
